# EUROPÄISCHE PATENTSCHRIFT

(11) **EP 0 696 901 B1**
(45) Veröffentlichungstag und Bekanntmachung des Hinweises auf die Patenterteilung: **19.05.1999**
(21) Anmeldenummer: 95910391.2
(22) Anmeldetag: 07.02.1995
(51) Int. Cl.: A61B 17/32

(54) **ENDOSKOPISCHE SCHNEIDVORRICHTUNG**
ENDOSCOPIC CUTTING DEVICE
DISPOSITIF DE COUPE ENDOSCOPIQUE

(30) Priorität: 07.02.1994 DE 4403602
(43) Veröffentlichungstag der Anmeldung: 21.02.1996
(73) Patentinhaber: Karl Storz GmbH & Co., 78532 Tuttlingen (DE)
(72) Erfinder: CUSCHIERI, Alfred, Dundee DD1 9SY (GB); FRANK, Tim, Dundee DD1 9SY (GB)
(74) Vertreter: Münich, Wilhelm, Dr.
(86) Internationale Anmeldenummer: DE9500166
(87) Internationale Veröffentlichungsnummer: WO9520918

(56) Entgegenhaltungen:
- EP-A- 0 569 256
- WO-A-91/02493
- DE-A- 2 160 466
- DE-A- 4 204 051
- US-A- 3 835 859
- US-A- 4 733 662
- US-A- 5 201 741

## Beschreibung

### Technisches Gebiet

Die Erfindung bezieht sich auf eine endoskopische Schneidvorrichtung zum Abtrennen von intrakorporalen Körperproben beispielsweise Organen oder Organteilen, gemäß dem Oberbegriff des Patentanspruchs 1.

Das Abtrennen und Zerschneiden von intrakorporalen Proben wie beispielsweise von Gewebe, Organen bzw. Organteilen, Tumoren, Gefäßen, um nur einige Beispiele für intrakorporale Proben zu nennen, ist bei endoskopischen Eingriffen bzw. Operationen sehr häufig erforderlich.

### Stand der Technik

Dementsprechend zahlreich sind die Vorschläge für endoskopische Schneidvorrichtungen:

Eine endoskopische Schneidvorrichtung gemäß dem Oberbegriff des Patentanspruchs 1 ist aus der DE-A-21 60 466 bekannt. Die aus dieser Druckschrift bekannte Exzisions-Zange weist eine langgestreckte flexible Röhre auf, aus der eine Drahtschleife herausschiebbar ist. Der distale Endteil besteht aus zwei durch einen Schlitz voneinander getrennten Schenkeln, so daß durch Verschieben der Drahtschleife eine gewisse Schneidwirkung erzielt wird. Es ist jedoch mit dieser bekannten Exzisions-Zange nicht möglich, Gewebeproben derart zu entnehmen, daß eine Zuordnung der Probenstücke zu der ganzen Probe möglich ist. Weiterhin geht aus der WO 92/11816 ein medizinisches Schneidinstrument hervor, das an seinem distalen Schaftende eine um seine Längsachse rotierbar gelagerte Schneidvorrichtung aufweist, die propellerartig die zu bearbeitenden Körperproben zerschneidet und auch zerkleinert.

In vorteilhafter Weise läuft der Zerkleinerungsvorgang innerhalb einer Hülle ab, so daß die zerkleinerten Probenstücke bequem durch den Durchführungskanal samt Hülle nach außen gebracht werden können.

Nachteilig ist jedoch, daß die derart zerkleinerten Probenstücke nur bedingt für eine exakte, histologische Untersuchung geeignet sind, zumal die ursprüngliche Gestalt des abgetrennten Körperteils durch die eher willkürliche Zerkleinerung praktisch gänzlich verloren geht.

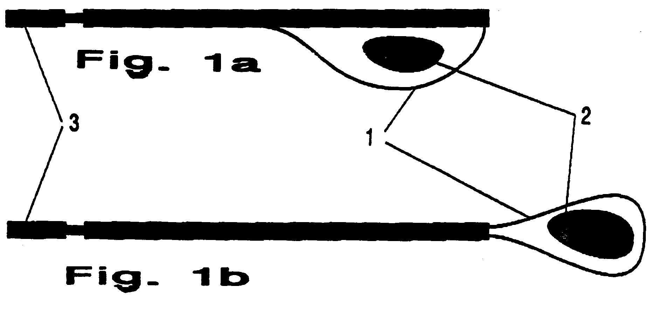

Desweiteren sind Schneidvorrichtungen zum Abtrennen intrakorporaler Körperproben bekannt, die schematisch in den Figuren 1a und 1b dargestellt sind. Es handelt sich hierbei um sogenannte Schlingenschneidvorrichtungen, an deren distalem Ende eine Drahtschlinge vorgesehen ist, die in ihrem Schlingendurchmesser variert werden kann.

Das Abtrennen von in der Konsistenz etwas stabileren Organteilen erfordert für das Erreichen sauberer Schnittflächen hohe Schneidekräfte. Dies wird am günstigsten dadurch ermöglicht, indem möglichst dünne Schneiddrähte verwendet werden. Dabei stellt sich jedoch das Problem, daß die Reiß- und Bruchfestigkeit derartiger Drähte mit abnehmenden Drahtdurchmesser stark zurückgeht. Insbesondere müssen die Drähte beim Zusammenziehen, d.h. während des Schneidvorganges, sehr hohen Krümmungen stand halten.

Oftmals werden dabei die Schneiddrähte im Zustand der geringsten Schlingenöffnung geknickt, mit der Folge, daß sie an dieser Stelle brechen.

### Darstellung der Erfindung

Der Erfindung liegt die Aufgabe zugrunde, eine endoskopische Schneidvorrichtung zum Abtrennen von Gewebe, wie Organen oder Organteilen, anzugeben, bei der das Abtrennen der zu entfernenden und zu untersuchenden Körperprobe und die zur Herausnahme der Probe erforderliche Zerkleinerung dieser Probe in einer kontrollierten Weise erfolgt, so daß eine histologische Untersuchung der Körperprobe im Ganzen, das heißt eine nachträgliche räumliche Zusammenfügung aller Probenstücke zu einer ganzen Probe, möglich sein soll.

Darüberhinaus soll die erfindungsgemäße Schneidvorrichtung so stabil sein, daß auch Proben abgetrennt werden können, die eine vergleichsweise hohe Konsistenz bzw. Festigkeit haben.

Eine erfindungsgemäße Lösung dieser Aufgabe ist im Patentanspruch 1 gekennzeichnet. Weiterbildungen der Erfindung sind Gegenstand der Ansprüche 2 folgende.

Erfindungsgemäß wird von einer endoskopischen Schneidvorrichtung zum Abtrennen von Gewebe, wie Organen, Organteilen oder dgl., mit einem Schneidwerkzeug, das am distalen Ende der Schneidvorrichtung angebracht ist, und das eine geradlinig ausgebildete Schneidklinge aufweist, die in etwa parallel zu einer Längseinheit angeordnet ist, die in ihrer Länge variierbar ist, und einer Betätigungseinheit, die zum Bedienen des Schneidwerkzeugs dient und die am proximalen Ende der Vorrichtung angebracht ist, ausgegangen, und diese Schneidvorrichtung dadurch weitergebildet, daß die Längseinheit zwei elastische Bänder aufweist, die beidseitig der Schneidklinge angeordnet sind, und daß die Bänder distalseitig fest an der Vorrichtung und proximalseitig an einer mit der Betätigungseinheit verbundenen Schubstange angebracht sind, so daß sie durch eine Bedienung der Betätigungseinheit in ihrer Länge variierbar sind, wobei im gelockerten Zustand die Bänder über der Schneidklinge eine Bucht bilden, die über das abzutrennende Organ oder Organteil gelegt werden kann, und im gestrafften Zustand die Bänder unterhalb und parallel zur Schneidklinge verlaufen, so daß das abzutrennende Gewebe gegen die Schneidklinge gedrückt und abgetrennt wird.

Eine weitere Ausführungsform ermöglicht die Herabsetzung der aufzuwendenden Schneidkraft dadurch, daß die Schneidklinge während des Straffens der Längseinheit gegen das abzutrennende Gewebe gedrückt und damit das Gewebe abgetrennt wird.

Im Unterschied zu den bekannten Schneidvorrichtungen weist die erfindungsgemäße Schneidvorrichtung eine feststehende Schneidklinge auf, gegen die die abzutrennende Körperprobe mit Hilfe der Längseinheit gedrückt wird.

Die Längseinheit besteht in vorteilhafter Weise aus zwei elastischen Bändern, die jeweils beidseitig neben der Schneidklinge im gespannten Zustand zu liegen kommen. Der Spannungszustand der Bänder kann über die Betätigungseinheit, die am proximalen Instrumentenende vorgesehen ist, variiert werden.

Die elastischen Bänder sind am distalen Ende des Instruments fest eingespannt und gleiten am anderen Ende des Schneidkopfes durch entsprechende Führungsaussparungen in das Innere des Instrumentenhalses und sind schließlich mit der Betätigungseinheit am proximalen Ende des gesamten Instruments verbunden.

Diese Anordnung gestattet es, im entspannten Zustand der elastischen Bänder eine Bucht zu bilden, die zum einen durch den bogenförmigen Bandverlauf und zum anderen durch die gerade Schneidklinge begrenzt ist. Durch diese Bucht kann die abzutrennende Körperprobe geführt werden.

Durch eine Sichtbeobachtung während des Operationsvorgangs ist es möglich, die Schnittebene an dem abzutrennenden Körperteil definiert zu setzen, und in einer kontrollierten Weise die Körperprobe gegen die geradlinige Schneide zu drücken. Hierzu wird die Betätigungseinheit am proximalen Instrumentenende derart bedient, so daß die parallel verlaufenden Bänder gestrafft werden.

Gekoppelt mit dem Vorgang der Bandstraffung ist es besonders vorteilhaft, wenn die Schneidklinge in ihrer Längsrichtung bewegbar geführt ist. Auf diese Weise überlagern sich der bloße Pressdruck des abzutrennenden Körperteiles gegen die Klinge mit der Schneidwirkung der Klinge, die durch ihre Hin- und Herbewegung erzeugt wird, so daß der jeweilige Körperteil leichter abgetrennt werden kann.

Ebenso ermöglicht eine weitere Ausführungsform die Herabsetzung der aufzuwendenden Schneidkraft dadurch, daß die Schneidklinge während des Straffens der Längseinheit gegen das abzutrennende Gewebe bewegbar ist.

In vorteilhafter Weise sind die Bänder als Rechtecksbänder bestehend aus einer Nickel-Titanlegierung ausgeführt und weisen eine typische Querschnittsgröße von 2 x 0,7 mm auf.

Mit Hilfe der erfindungsgemäßen Vorrichtung ist es unter Sichtbeobachtung mittels Endoskopen möglich, die Schnittflächen an der abgetrennten Körperprobe derart zu legen, daß diese in definierter Weise zerteilt wird, so daß die einzelnen zertrennten Körperprobenteile nach dem Ausbringen aus der Körperhöhle für eine histologische Untersuchung wieder zusammengesetzt werden können. Hierdurch ist es erstmalig möglich, genau nachzuvollziehen, an welchen Stellen in der zu untersuchenden Probe beispielsweise krebsartige Zellstrukturen gewachsen sind.

### Kurze Beschreibung der Zeichnung

Die Erfindung wird nachstehend ohne Beschränkung des allgemeinen Erfindungsgedankens anhand eines Ausführungsbeispiels unter Bezugnahme auf die Zeichnung exemplarisch beschrieben. Es zeigen:
- Fig. 1a, 1b: bekannte Schlingenschneidvorrichtungen,
- Fig. 2: das distale Ende eines Ausführungsbeispiels einer erfindungsgemäßen endoskopischen Schneidvorrichtung, und
- Fig. 3: eine schematische Seitengesamtansicht des dargestellten Ausführungsbeispiels.

### Darstellung eines Ausführungsbeispiels

Die Figuren 1a und 1b zeigen an sich bekannte endoskopische Schneidvorrichtungen, die einen Schneiddraht 1 aufweisen, der um eine abzutrennende Körperprobe gelegt wird und mit Hilfe eines Betätigungselementes 3 derart gestrafft bzw. gespannt wird, daß die abzutrennende Körperprobe 2 von dem restlichen Körperteil abgetrennt werden kann.

Fig. 1a zeigt eine Ausführungsform, die eine seitlich am distalen Ende des schaftförmig ausgeformten Instrumentes angeordnete Drahtschlinge aufweist.

Fig. 1b zeigt eine Variante, nämlich ein sog. Drahtschlingenschneidwerkzeug, an dem der Schneidedraht 1 in Verlängerung zum schaftförmigen Instrument an seiner distalen Spitze angeordnet ist.

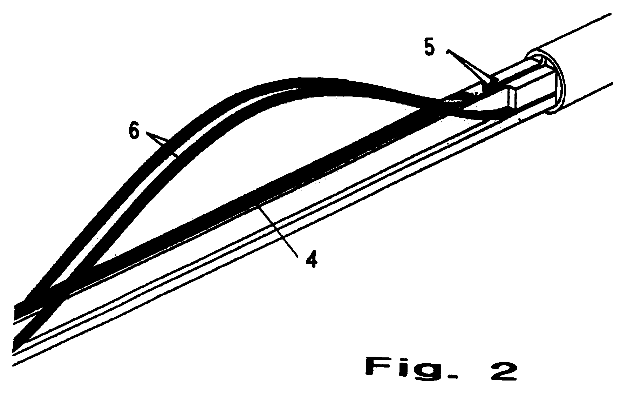

Fig. 2 zeigt das distale Ende eines Ausführungsbeispiels einer erfindungsgemäßen endoskopischen Schneidvorrichtung.

Das gezeigte Ausführungsbeispiel weist eine geradlinig ausgestaltete Schneidklinge 4 auf, die zwischen zwei Gehäuseplatten 5 versenkbar ist. Die Gehäuseplatten 5 dienen - wie weiter unten noch beschrieben wird - auch als Schubstangen.

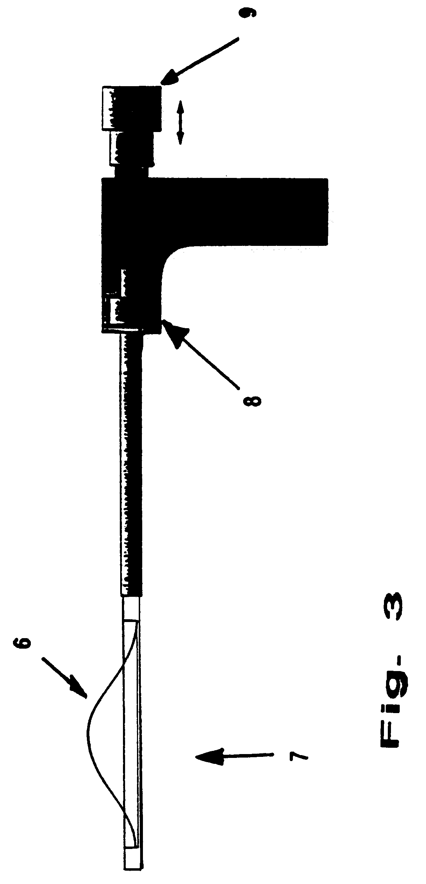

Wie Fig. 3 zeigt, ist die Schneidvorrichtung am distalen Ende 7 eines mit einem Schaft versehenen Instruments angebracht. Die elastischen Bänder 6 sind wie in Fig. 2 dargestellt, beidseitig parallel neben der Klinge 4 am distalen Ende der Schneidvorrichtung befestigt und an ihrem entgegengesetzten Ende der Schneidvorrichtung durch den Schaft über die Schubstangen 5 mit einer Betätigungsvorrichtung (siehe Fig. 3 derart verbunden, daß die elastisch ausgestalteten Bänder 6 gestrafft werden können. Im gestrafften Zustand liegen die Bänder 6 unterhalb des Niveaus der Schneidklinge 4, parallel zu ihr eng an den Gehäuseplatten 5 an. Somit ist gewährleistet, daß jegliches Material, das zwischen den Bändern 6 und der Schneidklinge 4 eingebracht worden ist vollkommen durchtrennt werden kann.

In Fig. 3 ist eine schematisierte Seitenansicht des gesamten Schneidinstrumentes dargestellt. Die endoskopische Schneidvorrichtung ist am distalen Ende 7 angebracht. Im Instrumentengehäuse ist das Betätigungselement 8 untergebracht, mit Hilfe dessen die elastischen Bänder 6 gestrafft bzw. lösbar sind. Während des Lösens mit dem Betätigungselement 8 nehmen die Bänder eine vorgeformte, bogenförmige, eine Bucht mit der Schneidklinge bildende Form an, deren Größe an die Organgröße der abzutrennden Körperproben anpassbar ist. Das Material, aus dem die Bänder bestehen ist bevorzugterweise eine Nickel-Titan-Legierung und weist einen Shape-Memory-Effekt auf. Am proximalen Ende des Schneidinstruments ist eine Bedieneinheit 9 vorgesehen, über die die Schneidklinge in Bewegung versetzt werden kann. Durch Bewegen der Schneidklinge können die Schneidkräfte während des Abtrennvorgang erheblich reduziert werden.

## Patentansprüche

1. Endoskopische Schneidvorrichtung zum Abtrennen von Gewebe, wie Organen, Organteilen oder dgl., mit
- einem Schneidwerkzeug, das am distalen Ende der Schneidvorrichtung angebracht ist, und das eine geradlinig ausgebildete Schneidklinge (4) aufweist, die in etwa parallel zu einer Längseinheit angeordnet ist, die in ihrer Länge variierbar ist, und
- einer Betätigungseinheit (9), die zum Bedienen des Schneidwerkzeugs dient und die am proximalen Ende der Vorrichtung angebracht ist,
dadurch **gekennzeichnet**, daß die Längseinheit zwei elastische Bänder (6) aufweist, die beidseitig der Schneidklinge angeordnet sind, und daß die Bänder distalseitig fest an der Vorrichtung und proximalseitig an einer mit der Betätigungseinheit (9) verbundenen Schubstange (5) angebracht sind, so daß sie durch eine Bedienung der Betätigungseinheit in ihrer Länge variierbar sind, wobei
- im gelockerten Zustand die Bänder über der Schneidklinge eine Bucht bilden, die über das abzutrennende Organ oder Organteil gelegt werden kann, und
- im gestrafften Zustand die Bänder unterhalb und parallel zur Schneidklinge verlaufen, so daß das abzutrennende Gewebe gegen die Schneidklinge gedrückt und abgetrennt wird.

2. Schneidvorrichtung nach Anspruch 1, dadurch **gekennzeichnet**, daß die Schneidklinge in Längsrichtung mittels einer Betätigungseinrichtung (9) bewegbar ist.

3. Schneidvorrichtung nach Anspruch 1 oder 2, dadurch **gekennzeichnet**, daß die Längseinheit aus einem Material mit shape-memory-Effekt besteht.

4. Schneidvorrichtung nach Anspruch 3, dadurch **gekennzeichnet**, daß die Längseinheit aus einer Nickel-Titan-Legierung besteht und eine Bandform mit einer rechteckigen Querschnittsgröße von 2 x 0.7 mm aufweist.

5. Schneidvorrichtung nach einem der Ansprüche 1 bis 4, dadurch **gekennzeichnet**, daß die Schneidklinge während des Straffens der Längseinheit gegen das abzutrennende Organ oder Organteil bewegbar ist.

## Claims

1. An endoscopic cutting device for severing tissue, such as organs, parts of organs or the like, having
- a cutting tool, which is attached at the distal end of said cutting device and which is provided with a straight cutting blade (4) which is disposed approximatel parallel to a longitudinal unit the length of which is variable, and
- an actuating unit (9) which is used to operate said cutting tool and which is attached at the proximal end of said device,
**characterized by** said longitudinal unit being provided with two elastic bands (6) which are disposed on both sides of said cutting blade and by said bands being firmly attached at their distal end to said device and at their proximal end to a slide rod (5) connected to said actuating unit (9) in such a manner that the length of said bands is variable by operating said actuating unit, with
- in a loose state said hand forming over said cutting blade a bay which can be placed over the to-be-severed organ or part of an organ, and
- in a tautened state said bands running below and parallel to said cutting blade in such a manner that the to-be-severed tissue is pressed against said cutting blade and is severed.

2. A cutting device according to claim 1,
**characterized by** said cutting blade being moveable in the longitudinal direction by means of an actuating device (9).

3. A cutting device according to one of the claims 1 or 2,
**characterized by** said longitudinal unit being composed of a material having a shape-memory effect.

4. A cutting device according to claim 3,
**characterized by** said longitudinal unit being composed of a nickel-titanium alloy and having a band-shaped form having a rectuangular cross section size of 2 x 0.7 mm.

5. A cutting device according to one of the claims 1 to 4,
**characterized by** said cutting blade being moveable against the to-be-severed organ or part of the organ during tautening of said longitudinal unit.

## Revendications

1. Dispositif de coupe endoscopique à détacher de tissu, comme des organes, des fragments d'organe, ou parties similaires, comprenant
- un outil tranchant disposé à l'extrémité distale du dispositif de coupe, et comprenant une lame de découpe (4) à configuration rectiligne, qui est disposée environ en paralléle à une unité longitudinale à longueur variable, et
- une unité de commande (9) qui sert à manoeuvrer l'outil tranchant et qui est disposé à l'extrémité proximale du dispositif,
**caractérisé** en ce que ladite unité longitudinale comprend deux rubans élastiques (6) disposés des deux côtés de la lame de découpe, et en ce que lesdits rubans sont fixés, du côté distal, a un dispositif et du côté proximale à un lever de commande (5) relié à ladite unité de commande (9), de façon que leur longueur est variable par un actionnement de ladite unité de commande,
- les rubans, en état relâché, formant une baie via la lame de découpe, qui peut être placée sur l'organe ou le fragment d'organe à détacher,
- et les rubans, en état tendu, s'étendant au dessous de et en paralléle à la lame de découpe de façon que le tissu à détacher soit pressé contre la lame de découpe pour sa séparation.

2. Dispositif de coupe selon la revendication 1,
**caractérisé** en ce que ladite lame de découpe est mobile moyennant un moyen de commande (9) le long de son orientation longitudinale.

3. Dispositif de coupe selon la revendication 1 ou 2,
**caractérisé** en ce que ladite unité longitudinale est faite en un matériau à effet de mémoire de forme, dit shape memory.

4. Dispositif de coupe selon la revendication 3,
**caractérisé** en ce que ladite unité longitudinale est faite en un alliage nickel-titane et présente une forme de ruban rectangulaire à une largeur de 2 x 0,7 mm en coupe transversale.

5. Dispositif de coupe selon une quelconque des revendications 1 à 4,
**caractérisé** en ce que ladite lame de découpe est mobile au cours de l'opération de tension de ladite unité longitudinale contre l'organe ou la partie d'un organe à détacher.
